# EUROPÄISCHE PATENTSCHRIFT

(11) **EP 1 694 318 B1**
(45) Veröffentlichungstag und Bekanntmachung des Hinweises auf die Patenterteilung: **14.03.2007**
(21) Anmeldenummer: 04803781.6
(22) Anmeldetag: 13.12.2004
(51) Int. Cl.: A61K 31/135, A61P 25/00, A61P 43/00

(54) **(S)-2-N-PROPYLAMINO-5-HYDROXYTETRALIN ALS D3-AGONISTISCHES THERAPEUTIKUM**
(S)-2-N-PROPYLAMINO-5-HYDROXYTETRALIN AS A D3-AGONIST
(S)-2-N-PROPYLAMINO-5-HYDROXYTETRALINE UTILISEE COMME AGENT THERAPEUTIQUE AYANT UN EFFET AGONISTE SUR LE RECEPTEUR D3

(30) Priorität: 18.12.2003 DE 10359528
(43) Veröffentlichungstag der Anmeldung: 30.08.2006
(73) Patentinhaber: SCHWARZ PHARMA AG, 40789 Monheim/Rhld. (DE)
(72) Erfinder: SCHELLER, Dieter, 41470 Neuss (DE); HANSEN, Klaus, 41516 Grevenbroich-Münchrath (DE)
(74) Vertreter: HOFFMANN EITLE
(86) Internationale Anmeldenummer: PCT/EP2004/014143
(87) Internationale Veröffentlichungsnummer: WO 2005/058296

(56) Entgegenhaltungen:
- EP-A- 0 026 848
- WO-A-94/26703
- WO-A-03/028725
- WO-A-03/092677
- US-A- 4 465 692
- SONESSON, CLAS ET AL: "Synthesis and Evaluation of Pharmacological and Pharmacokinetic Properties of Monopropyl Analogs of 5-, 7-, and 8-[[(Trifluoromethyl)sulfonyl]oxy]-2- aminotetralins: Central Dopamine and Serotonin Receptor Activity" JOURNAL OF MEDICINAL CHEMISTRY , 38(8), 1319-29 CODEN: JMCMAR; ISSN: 0022-2623, 1995, XP002325503
- HACKSELL, ULI ET AL: "N-Alkylated 2-aminotetralins: central dopamine-receptor stimulating activity" JOURNAL OF MEDICINAL CHEMISTRY , 22(12), 1469-75 CODEN: JMCMAR; ISSN: 0022-2623, 1979, XP002325504 in der Anmeldung erwähnt
- TIMMERMAN, WIA ET AL: "Microdialysis and striatal dopamine release: stereoselective actions of the enantiomers of N-0437" EUROPEAN JOURNAL OF PHARMACOLOGY , 162(1), 143-50 CODEN: EJPHAZ; ISSN: 0014-2999, 1989, XP002325505
- WIKSTROEM, HAAKAN ET AL: "Resolved monophenolic 2-aminotetralins and 1,2,3,4,4a,5,6,10b- octahydrobenzo[f]quinolines: structural and stereochemical considerations for centrally acting pre- and postsynaptic dopamine-receptor agonists" JOURNAL OF MEDICINAL CHEMISTRY , 28(2), 215-25 CODEN: JMCMAR; ISSN: 0022-2623, 1985, XP002325506
- SONESSON C ET AL: "Orally active central dopamine and serotonin receptor ligands: 5-, 6-, 7-, and 8-[[trifluoromethyl)sulfonyl]oxy]-2-(di-n- propylamino)tetral ins and the formation of active metabolites in vivo." JOURNAL OF MEDICINAL CHEMISTRY. 29 OCT 1993, Bd. 36, Nr. 22, 29. Oktober 1993 (1993-10-29), Seiten 3409-3416, XP002325507 ISSN: 0022-2623

## Beschreibung

Dopamin ist ein wesentlicher Neurotransmitter des zentralen Nervensystems. Seine Wirkung vermittelt Dopamin durch Bindung an fünf verschiedenen Dopaminrezeptoren. Diese lassen sich aufgrund ihrer Morphologie und ihrer Art der Signalübertragung in die Klassen "D1-like" (D1 und D5) sowie "D2-like" (D2-, D3- und D4-Rezeptoren) einteilen.

Der D3-Rezeptor wurde zum erstenmal von Sokoloff kloniert (Nature 347, 1990, 146) und wird vor allem im limbischen System, in dem emotionale und kognitive Vorgänge gesteuert werden, exprimiert. Etwas geringer ausgeprägt findet er sich im striatalmotorischen Bereich, wo er der Feinregulation von Bewegungsabläufen dient (Joyce, Pharmacol Ther. 90, 2001, 231-259). Der D3-Rezeptor gilt neuerdings als vielversprechendes Target für die Entwicklung von Wirkstoffen zur Behandlung verschiedener psychiatrischer und motorischer Erkrankungen.

D3-Agonisten können somit wertvolle Therapeutika zur Behandlung verschiedener Arten von Depressionen, krankhafter Angstzustände, sexueller Dysfunktion, Glaukoma, kognitiver Störungen, Restless Leg Syndrom, Hyperaktivitätssyndrom (ADHS), Hyperprolaktinämie, Hyperprolaktinom, Essstörungen, Parkinson-assoziierter Bewegungsstörungen, DOPA- und Neuroleptika-induzierter Bewegungsstörungen, z.B. Akathisie, Rigor, Dystonie und Dyskinesien sowie von Kokain-, Alkohol-, Opiat- und Nikotinsucht, Galactorrhoe und Akromegalie darstellen.

Ferner haben D3-Agonisten neuroprotektives Potenzial bei der Behandlung und Prophylaxe neurodegenerativer Erkrankungen (Pulvirenti, L. et al. *Trends Pharmacol. Sci.* 2002, *23,* 151-153; Joyce, Pharmacology and Therapeutics 90, 2001, 231; EP 988 296; WO 03/29233; WO 93/23035).

Es besteht daher ein Bedarf an hochaffinen D3-Agonisten mit möglichst großer funktioneller Selektivität im Verhältnis zu "D1-like"-Rezeptoren und mit signifikanter Selektivität im Verhältnis zu den übrigen "D2-like"-Rezeptoren.

Es wurde nun überraschend festgestellt, dass (S)-2-N-Propylamino-5-hydroxytetralin die gewünschten Eigenschaften hat.

Razemisches 2-N-Propylamino-5-hydroxytetralin ist aus der Literatur bekannt.

Hacksell et al (J Med Chem 22, 1979, 1469) untersuchten verschiedene N-alkylierte 2-Aminotetraline im Hinblick auf ihre Dopaminrezeptor-stimulierende Aktivität. Für das razemische 2-N-Propylamino-5-hydroxytetralin wird eine gewisse dopaminerge Aktivität nachgewiesen. Allerdings ist die agonistische Aktivität der Substanz mit einem ED₅₀ von 40 nM/kg nur mäßig und die AUC und die Halbwertszeit sind im Vergleich zu den anderen untersuchten Verbindungen kurz. Als aktivste und für die beabsichtigte orale Gabe geeignete Verbindungen erwiesen sich Aminotetraline mit N,N-Dialkylierung.

Beaulieu et al (Eur J Pharmacol 105, 1984, 15) untersuchten N,N-disubstituierte 2-Aminotetraline im Hinblick auf ihre D2-stimulierende Aktivität. Das razemische 2-N-Propylamino-5-hydroxytetralin erwies sich als moderat aktiv, während N,N-dialkylierte 2-Amino-5-Hydroxy Derivate, wie das N-0437 (razemisches Rotigotin) eine signifikant höhere Aktivität zeigte. Schlußfolgerungen zum etwaigen therapeutischen Potenzial von 2-N-Propylamino-5-hydroxytetralin werden nicht gezogen.

WO-A-03/092677 offenbart die Verwendung von Rotigotin zur Behandlung des "Restless Leg Syndroms" und des Parkinson Syndroms. Pharmazeutische Zusammensetzungen und eine transepikutane Verabreichung werden ebenfalls offenbart.

Seiler et al (J Med Chem 29,1986, 912) offenbaren 2-N-Propylamino-5-hydroxytetralin als Edukt zur Synthese N-dialkylierter Verbindungen. Eine biologische Wirksamkeit von 2-N-Propylamino-5-hydroxytetralin wird nicht beschrieben.

Swart et al (Toxikology Methods 3,1993, 279) beschreiben das Razemat von 2-N-Propylamino-5-hydroxytetralin als Rotigotin-Metabolit mit schwacher dopaminerger Wirkung. Rotigotin [5,6,7,8-Tetrahydro-6-[propyl[2-(2-thienyl)ethyl]amino]-1-naphthalenol] ist ein Beispiel für einen Dopamin-Rezeptor Agonisten mit D2/D3-agonistischer Aktivität. Im Verhältnis zum Rotigotin, dass mit einem K_{d}-Wert von 5 nM an eine dopaminrezeptorreiche Membranfraktion bindet, weist 2-N-Propylamino-5-hydroxytetralin einen deutlich höheren K_{d}-Wert von 1,3 µM auf. Die Autoren kommen zu dem Schluß, dass die N-dealkylierten Metaboliten von Rotigotin eine zu schwache dopaminerge Aktivität besitzen, um therapeutische Relevanz zu haben.

Swart et al (J. Analytical Toxicology 18,1994, 71) offenbaren das (S)-Enantiomer von 2-N-Propylamino-5-hydroxytetralin als Metabolit von Rotigotin. Eine biologische Wirksamkeit wird nicht beschrieben.

Sonesson et al (J Med Chem 38, 1995, 1319) untersuchten die biologische Wirksamkeit von Monopropylanaloga von {[(Trifluormethyl)sulfonyl]oxy}-2-Aminotetralinen. Die Enantiomere von 2-N-Propylamino-5-hydroxytetralin werden dabei als Synthesezwischenprodukt offenbart, jedoch biologisch nicht charakterisiert.

EP 026 848, EP 717 620, WO 94/26703 und WO 01/38321 offenbaren 2-N-Propylamino-5-hydroxytetralin als Edukt zur Synthese N-dialkylierter bzw. sulfonierter Aminotetraline. Die medizinische Verwendung von 2-N-Propylamino-5-hydroxytetralin wird nicht vorgeschlagen.

Van Vliet et al (J Med Chem 39, 1996, 4233) untersuchen die Eignung von Kompetitionstests mit D2L-Agonisten und D2L-Antagonisten zur Vorhersage der Dopamin-Rezeptorsubtyp Selektivität. Hierzu werden Aminotetraline im Hinblick auf ihre D3-Selektivität und ihre potentielle Eignung als Antipsychotika untersucht. Im Rahmen dieser Untersuchungen wurde razemisches 2-N-Propylamino-5-hydroxytetralin sowie 27 weitere Substanzen eingesetzt. Funktionelle Daten zur (ant)agonistischen Aktivität der verwendeten Substanzen wurden nicht erhoben. Die medizinische Verwendung von 2-N-Propylamino-5-hydroxytetralin wird nicht vorgeschlagen. Stattdessen kommen die Autoren auf Seite 4236 zur Schlußfolgerung, daß mit Ausnahme von Verbindung (+)25 keine der eingesetzten Substanzen dem pharmakologischen Wunschprofil eines D3-selektiven Antipsychotikums entspricht.

Zusammengefasst ist razemisches 2-N-Propylamino-5-hydroxytetralin aus dem Stand der Technik als unselektiver, mäßig aktiver Dopaminagonist mit geringer Halbwertszeit bekannt. Obwohl eine gewisse dopaminerge Aktivität des razemischen 2-N-Propylamino-5-hydroxytetralins schon seit 1979 bekannt ist (siehe Hacksell et al, supra), wurde eine medizinische Verwendung dieser Substanz nicht beschrieben und auch nicht vorgeschlagen. Im Gegenteil kommen Swart et al zu dem Schluß, dass die N-dealkylierten Metaboliten von Rotigotin eine zu schwache dopaminerge Aktivität besitzen, um therapeutische Relevanz zu haben (Tox Meth 3, 1993, Seite 289, letzter Absatz).

Für den Fachmann ergab sich somit keine Motivation, eine Enantiomerentrennung dieser offenbar therapeutisch ungeeigneten Substanz vorzunehmen und die einzelnen Enantiomere auf ihr therapeutisches Potenzial zu prüfen.

Es war daher überraschend, dass das reine (S)-Enantiomer von 2-N-Propylamino-5-hydroxytetralin eine ausgeprägte Affinität zu und eine bemerkenswerte funktionelle Selektivität für den D3-Rezeptor sowie eine rein agonistische Aktivität aufweist, die die Substanz zu einem wertvollen Kandidaten bei der Behandlung von Dopaminmangelerkrankungen macht. Dieses therapeutisch attraktive Profil des reinen (S)-Enantiomers wurde in den bisherigen Studien mit 2-N-Propylamino-5-hydroxytetralin nicht identifiziert.

Tatsächlich bindet das (S)-Enantiomer von 2-N-Propylamino-5-hydroxytetralin mit einem Ki-Wert von 7,6 nM an den D3-Rezeptor. Im Vergleich dazu ist die Bindung gegenüber anderen Dopaminrezeptorsubtypen weit schwächer ausgeprägt. Insgesamt ergibt sich in den Rezeptorbindungstests eine Selektivität D3/D1 und D3/D5 von >1000 und von D3/D2 von ca. 40 (Tabelle 1).

**Tabelle 1: IC₅₀-Werte von (S)-2-N-Propylamino-5-hydroxytetralin an Rezeptorsubtypen**

| Rezeptor | Ki [nM] |
|---|---|
| Dopamine D₁ (h) | >10000 |
| Dopamine D_{2S} (h) | 290 |
| Dopamine D₃ (h) | 7.6 |
| Dopamine D_{4.2} (h) | 30 |
| Dopamine D_{4.4} (h) | 27 |
| Dopamine D_{4.7} (h) | 46 |
| Dopamine D₅ (h) | 2000 |

Ferner wurde in funktionellen Tests gefunden, daß die Aktivität von (S)-2-N-Propylamino-5-hydroxytetralin rein agonistisch ist und eine stark ausgeprägte funktionelle D3-Selektivität im Vergleich zum D1-Rezeptor sowie eine signifikante Selektivität im Vergleich zum D2-Rezeptor vorliegt (Tabelle 2).

**Tabelle 2: EC₅₀-Werte von (S)-2-N-Propylamino-5-hydroxytetralin an Rezeptorsubtypen**

| Rezeptorsubtyp | EC₅₀ (nM) |
|---|---|
| D1 | 1129 |
| D2L | 2,7 |
| D3 | 0,67 |
| D4.4 | 23,4 |
| D5 | 1310 |

Im Vergleich zu (S)-2-N-Propylamino-5-hydroxytetralin weisen die strukturell sehr ähnlichen Verbindungen AJ 76 und UH 232 (Hackling und Stark, ChemBioChem 3, 2002, 947) eine geringere D3-Präferenz auf. Weiterhin war es überraschend festzustellen, daß (S)-2-N-Propylamino-5-hydroxytetralin D2/D3-agonistische Wirkung hat, während das strukturell eng verwandte AJ 76 als reiner Antagonist beschrieben ist. Das sich daraus ergebende therapeutische Profil von (S)-2-N-Propylamino-5-hydroxytetralin weicht daher erheblich von dem des strukturell ähnlichen AJ 76 ab.

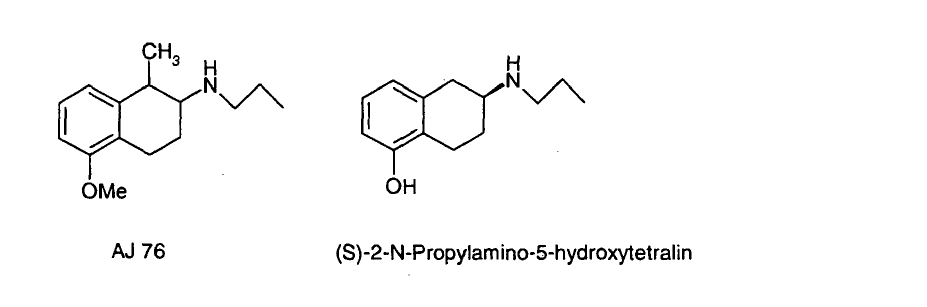

Im Vergleich zum Rotigotin, aus dem das (S)-2-N-Propylamino-5-hydroxytetralin in geringen Mengen metabolisch hervorgeht, zeigt (S)-2-N-Propylamino-5-hydroxytetralin die gleiche agonistische Effektivität (EC₅₀) am D3-Rezeptor, aber 564-fach bzw. 385fach geringere Affinitäten zum D1 und D5-Rezeptor und damit eine höhere Selektivität für D3 im Verhältnis zu diesen Rezeptoren.

Mit (S)-2-N-Propylamino-5-hydroxytetralin wurde somit ein Aminotetralin als hochaffiner D3-Agonist mit großer funktioneller Selektivität im Verhältnis zu dopaminergen D1- und D5-Rezeptoren, beträchtlicher Selektivität zum D4.4-Rezeptor und signifikanter Selektivität im Verhältnis zum D2L-Rezeptor für die Therapie von Erkrankungen, die auf die Therapie von Dopamin oder Dopaminagonisten ansprechen, zur Verfügung gestellt.

Ein Gegenstand der vorliegenden Erfindung ist somit ein Arzneimittel enthaltend 2-N-Propylamino-5-hydroxytetralin oder dessen pharmazeutisch akzeptable Salze und Prodrugs, wobei 2-N-Propylamino-5-hydroxytetralin bevorzugt als reines (S)-Enantiomer vorliegt.

Unter dem Begriff "reines (S)-Enantiomer" wird in dieser Erfindung verstanden, dass der Anteil des (R)-Enantiomeren im Arzneimittel bevorzugt mit einem Anteil von < 10 Mol%, besonders bevorzugt mit einem Anteil von < 2 Mol% und ganz besonders bevorzugt mit einem Molanteil von < 1 %, bezogen auf die Gesamtmenge 2-N-Propylamino-5-hydroxytetralin im Arzneimittel vorliegt.

Der Begriff "pharmazeutisch akzeptable Salze" umfasst insbesondere nicht-toxische Additionssalze von 2-N-Propylamino-5-hydroxytetralin mit organischen oder anorganischen Säuren sowie deren Hydrate und Solvate. Beispiele für anorganische Säuren schließen HCl, HBr, Schwefelsäure, schweflige Säure, phosphorige Säure und Phosphorsäure ein. Organische Säuren schließen Essigsäure, Propionsäure, Brenztraubensäure, Buttersäure, α-, β- oder γ-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäure, Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Äpfelsäure, D-Äpfelsäure, D,L-Äpfelsäure, Oxalsäure, Malonsäure, Bernsteinsäure, Maleinsäure, Oxalessigsäure, Glutarsäure, Hydroxyglutarsäure, Ketoglutarsäure, Adipinsäure, Ketoadipinsäure, Pimelinsäure, Glutaminsäure, Asparaginsäure, Phthalsäure, Propantricarbonsäure, Zitronensäure, Isozitronensäure, Methansulfonsäure, Tuolsulfonsäure und Trifluormethansulfonsäure ein.

Die Herstellung von (S)-2-N-Propylamino-5-hydroxytetralin kann erfolgen wie in der Literatur beschrieben (siehe Hacksell et al, J Med Chem 22,1979,1469; Sonesson, J Med Chem 38, 1995, 1319; US 5,442,117). Die Herstellung der Prodrugs gemäß Anspruch 1 durch Reaktion von 2-N-Propylamino-5-hydroxytetralin mit entsprechenden reaktiven Vorstufen wie Säurechloriden, Säureanhydriden, Carbamoylchloriden etc. ist dem Fachmann auf dem Gebiet der klinischen Chemie bekannt. Entsprechende Vorschriften lassen sich der einschlägigen Fachliteratur entnehmen. Beispiele für Literaturstellen zur Herstellung von Prodrugs sind Bundgaard: Design of Prodrugs, Elsevier, Amsterdam, 1985; Higuchi und Stella: Pro-drugs as novel drug delivery systems in American Chemical Society, Washington DC, 1975; Sloan: Prodrugs - Topical and Ocular Drug Delivery, Ed: M. Dekker, 1992; Roche: Design of biopharmaceutical properties through prodrugs and analogs, Washington, DC, 1977.

Die grundsätzliche Eignung eines 2-N-Propylamino-5-hydroxytetralin-Derivats gemäß Anspruch 1 als Prodrug kann beispielsweise bestimmt werden, indem die jeweilige Verbindung unter definierten Bedingungen mit einem Enzymcocktail, einem Zellhomogenisat oder einer enzymhaltigen Zellfraktion inkubiert wird und das entstehende 2-N-Propylamino-5-hydroxytetralin gemessen wird. Eine geeignete Enzymmischung ist beispielsweise enthalten in der S 9-Leberpräparation der Firma Gentest, Woburn, Ma, USA.

Alternativ kann eine Inkubation mit Frischblut oder Plasma oder aber eines Homogenates der Unterhaut erfolgen, um eine leberunabhängige Metabolisierung der Prodrugs zur Wirkkomponente zu demonstrieren. Für transdermale Applikation ist eine in vitro Untersuchung der Permeation an exzidierter Haut notwendig. Der endgültige Nachweis der Eignung und potentiellen Wirksamkeit in den Krankheitsmodellen erfolgt durch eine Bestimmung des aus dem Prodrug gebildeten 2-N-Propylamino-5-hydroxytetralins im Plasma.

In-vivo sollte ein Prodrug soviel (S)-2-N-Propylamino-5-hydroxytetralin freisetzen, dass eine therapeutisch effektive steady-state Konzentration von (S)-2-N-Propylamino-5-hydroxytetralin im Plasma erreicht wird. Als therapeutisch effektive Konzentrationen werden dabei im allgemeinen (S)-2-N-Propylamino-5-hydroxytetralin-Konzentrationen zwischen 0.02 und 100 ng/mL, bevorzugt zwischen 0.05 ng und 50 ng/mL und besonders bevorzugt zwischen 0.1 und 40 ng/mL Plasma angesehen.

Ein weiterer Gegenstand der Erfindung ist daher ein Arzneimittel, enthaltend ein Prodrug der allgemeinen Formel I: 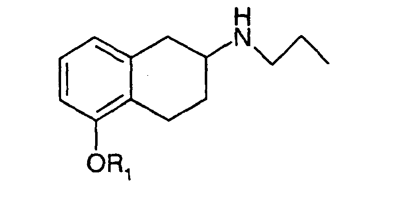 wobei R1 ausgewählt ist aus der Gruppe Acyl, Alkoxycarbonyl, Cycloalkoxycarbonyl, Aryloxycarbonyl, Aralkoxycarbonyl, Acetal, Ketal, -C(O)NR2R3, -C(O)NHR2, -P(O₂H)OR2,- P(O₂H)R2
wobei R2 und R3 jeweils ausgewählt sind aus H, C1-6 Alkyl, C3-10 Cycloalkyl, Benzyl oder Phenyl, und wobei die Verbindung der Formel I als reines (S)-Enantiomer vorliegt.

Bevorzugt ist R1 dabei ausgewählt ist aus der Gruppe C1-6 Alkylcarbonyl, C3-10 Cycloalkylcarbonyl, Benzoyl, -C(O)NR2R3 und -C(O)NHR2.

"Alkyl" kann eine verzweigte oder unverzweigte Alkylgruppe sein, die vorzugsweise 1 bis 10 C-Atome, besonders bevorzugt 1 bis 6 C-Atome und ganz besonders bevorzugt 1, 2 oder 3 C-Atome aufweist,. z.B. Methyl, Ethyl, n-Propyl, iso-Propyl, n-Butyl, iso-Butyl, s-Butyl, t-Butyl, n-Pentyl, iso-Pentyl, Neopentyl, t-Pentyl, 1-Methylbutyl, 2-Methylbutyl, 1-Ethylpropyl, 1,2-Dimethylpropyl und n-Hexyl. Alkylgruppen können zusätzlich mit einem oder mehreren Substituenten substituiert sein, beispielsweise mit Halogen.

"Cycloalkyl" ist eine Alkylgruppe, die nur aus reinen ringbildenden C-Atomen bestehen kann oder optional weitere verzweigende C-Atome tragen kann. Bevorzugte Kettenlängen sind 3-10, besonders bevorzugt 4-8 oder 4-6 C-Atome.

"Alkoxy" ist die Gruppe -O-Alkyl, worin Alkyl vorzugsweise aus den oben für "Alkyl" angegebenen Gruppen ausgewählt ist. Bevorzugt ist Alkyloxy eine C1-C6-Alkyloxygruppe, besonders bevorzugt eine C1-3 Alkyloxygruppe.

"Aryl" ist bevorzugt Phenyl. Phenyl kann gegebenenfalls zusätzlich in einer oder mehreren Positionen substituiert sein, z.B. mit Alkoxy, Alkyl, Halogen oder Nitro.

"Aralkyl" ist die Gruppe -Alkyl-Aryl, worin Alkyl und Aryl vorzugsweise aus den oben für "Alkyl" bzw. "Aryl" angegebenen Gruppen ausgewählt sind. "Aralkyl" ist bevorzugt Benzyl.

"Acyl" umfasst insbesondere die Gruppen -C(O)-Alkyl ("Alkylcarbonyl"), -C(O)-Cycloalkyl ("Cycloalkylcarbonyl"), -C(O)-Aryl ("Arylcarbonyl") und C(O)-Alkyl-Aryl ("Aralkylcarbonyl"), worin "Alkyl", "Cycloalkyl", "Aryl" und "Aralkyl" vorzugsweise aus den oben für "Alkyl" "Cycloalkyl", "Aryl" und "Aralkyl" angegebenen Gruppen ausgewählt sind, wobei -C(O)-C1-C6-Alkyl und C(O)-Phenyl besonders bevorzugt werden. Acyl ist beispielsweise Acetyl, Propionyl, Butyryl oder -C(O)-Phenyl ("Benzoyl").

"Alkoxycarbonyl" ist die Gruppe -C(O)-O-Alkyl, worin "Alkyl" vorzugsweise aus den oben für "Alkyl" angegebenen Gruppen ausgewählt ist. Bevorzugt ist Alkoxycarbonyl eine C1-C6-Alkoxycarbonylgruppe.

"Cycloalkoxycarbonyl" ist die Gruppe -C(O)-O-Cycloalkyl, worin "Cycloalkyl" vorzugsweise aus den oben für "Cycloalkyl" angegebenen Gruppen ausgewählt ist.

"Aryloxycarbonyl" ist die Gruppe -C(O)-O-Aryl, worin "Aryl" vorzugsweise aus den oben für "Aryl" angegebenen Gruppen ausgewählt ist.

"Aralkoxycarbonyl" ist die Gruppe -C(O)-O-Aralkyl, worin "Aralkyl" vorzugsweise aus den oben für "Aralkyl" angegebenen Gruppen ausgewählt ist.

"Ketal" ist insbesondere die an das phenolische Sauerstoffatom gebundene Gruppe - CR'R"-O-Alkyl oder -CR'R"-O-Aryl, worin "Alkyl" und "Aryl" vorzugsweise aus den oben für "Alkyl" und "Aryl" angegebenen Gruppen ausgewählt sind und worin R' und R" unabhängig voneinander für Alkyl oder Arylgruppen stehen. "Acetal" unterscheidet sich von "Ketal" dadurch, dass bei Acetal der Substituent R' ein Wasserstoff ist.

"Halogen" ist bevorzugt Fluor, Chlor, Brom oder Iod.

Ein weiterer Gegenstand der Erfindung ist die Verwendung von 2-N-Propylamino-5 hydroxytetralin, insbesondere als reines (S)-Enantiomer, dessen pharmazeutisch akzeptables Salze oder Prodrugs gemäß Anspruch 1 zur Herstellung eines Arzneimittels zur Behandlung oder Prophylaxe einer Erkrankung ausgewählt aus der Gruppe der Kokain-, Alkohol-, Opiat- und Nikotinsucht; neurodegenerativer Störungen, insbesondere Morbus Parkinson; sexuelle Dysfunktion, insbesondere männliche erektile Dysfunktion; Depression, insbesondere endogene monophasische Depression ("major depression"); Hyperprolaktinämie; Hyperprolaktinom; Glaucoma; kognitive Störungen; Restless Leg Syndrom; Hyperaktivitätssyndrom (ADHS); Galactorrhoe; Akromegalie; Parkinson-assoziierte Bewegungsstörungen, z.B. Rigor, Dystonie und Dyskinesie; L-DOPA-induzierte Störungen, idiopathische Dystonien, insbesondere Segawa-Syndrom; Neuroleptika-induzierte (tardive) Dyskinesie, Dystonie und Akathisie sowie das Parkinson-Plus Syndrom.

Unter "Opiaten" werden in dieser Patentanmeldung sowohl natürlich vorkommende Opiate, wie das Morphium, als auch synthetische Opiate, wie das Heroin subsumiert.

Ferner können die Arzneimittel zum Medikamenten-unterstützten Abstillen nach Schwangerschaften eingesetzt werden.

Insbesondere sind die erfindungsgemäßen Verbindungen zur Herstellung eines Arzneimittels zur Behandlung L-DOPA-sensitiver Bewegungsstörungen geeignet. Solche Bewegungsstörungen können beispielsweise Dyskinesien, Dystonien, Rigor und Tremor sein. Unter dem Begriff "L-DOPA-sensitiv" wird dabei verstanden, dass die Bewegungsstörung günstig durch Gabe von Arzneimitteln beeinflusst werden kann, die die dopaminerge Signalübertragung beeinflussen. Ein typisches Beispiel hierfür ist das Segawa-Syndrom, eine idiopathische Dystonie, bei der das Ansprechen auf L-DOPA als diagnostisches Kriterium genutzt werden kann. Andere Beispiele für L-DOPA-sensitive Störungen sind Morbus Parkinon-assoziierte, oder L-DOPA oder Neuroleptika-induzierte Bewegungsstörungen sowie das Restless Leg Syndrom.

Morbus Parkinson-assoziierte oder L-DOPA- oder Neuroleptika-induzierte Bewegungsstörungen sind beispielsweise Dyskinesien, Dystonien und Gangstörungen ("Freezing"). Unter einer L-DOPA-Therapie tritt zudem regelmäßig das sogenannte "wearing off" Phänomen auf, dass heißt ein Wirkverlust von L-DOPA, der durch Monotherapie oder Kombinationstherapien mit geeigneten D3-Dopaminagonisten gelindert oder verlangsamt werden kann.

Eine bevorzugte Verwendung von (S)-2-N-Propylamino-5-hydroxytetralin,betrifft somit die Herstellung eines Arzneimittels zur Behandlung von Bewegungsstörungen, wie zum Beispiel Dyskinesien, Dystonien und Gangstörungen, die spontan im Zuge von Parkinson-Erkrankungen auftreten können, aber auch Medikamenten-induziert sein können. Unter den Medikamenten-induzierten Bewegungsstörungen, wie Dyskinesien und Dystonien sind insbesondere solche zu nennen, die durch L-DOPA oder Dopaminantagonisten induziert wurden.

Schließlich können die erfindungsgemäßen Arzneimittel in Abhängigkeit von der zu behandelnden Erkrankung auch als Kombinationspräparat zur gleichzeitigen oder sequentiellen Gabe ausgebildet sein.

Beispielsweise kann eine Verkaufseinheit, die eine zur Behandlung der Parkinson-Erkrankung enthaltende L-DOPA Medikation enthält, auch eine pharmazeutische Zusammensetzung umfassen, die (S)-2-N-Propylamino-5-hydroxytetralin oder dessen pharmazeutisch akzeptable Salze und Prodrugs gemäß Anspruch 1 enthält. Dabei können L-DOPA und die erfindungsgemäßen Verbindungen in der gleichen pharmazeutischen Formulierung, z.B. in einer Kombinationstablette, oder auch in unterschiedlichen Applikationseinheiten vorliegen, z.B. in Form zweier separater Tabletten oder in zwei unterschiedlichen Verabreichungsformen, z.B: als orale L-DOPA Medikation und als transdermale oder transmukosale (S)-2-N-Propylamino-5-hydroxytetralin Formulierung. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Eine Ausführungsform der Erfindung betrifft daher ein Arzneimittel, das L-DOPA oder ein Neuroleptikum sowie (S)-2-N-Propylamino-5-hydroxytetralin oder dessen pharmazeutisch akzeptable Salze und Prodrugs gemäß Anspruch 1 zur gleichzeitigen oder zeitlich aufeinanderfolgenden Verabreichung an den Patienten enthält.

Üblicherweise bestehen die erfindungsgemäßen Arzneimittel aus einer pharmazeutischen Zusammensetzung, die neben (S)-2-N-Propylamino-5-hydroxytetralin oder dessen pharmazeutisch akzeptable Salze und Prodrugs gemäß Anspruch 1 mindestens einen pharmazeutisch annehmbaren Träger oder Hilfsstoff enthält.

Dabei kann die pharmazeutische Formulierung in Abhängigkeit vom beabsichtigten Applikationsweg unterschiedlich ausgestaltet sein. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe, wie Füllstoffe, Sprengmittel, Bindemittel, Gleitmittel, Stabilisatoren, Aromastoffe, Antioxidantien, Konservierungsmittel, Dispersions- oder Lösungsmittel, Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Deutscher Apotheker Verlag, 1991) und Remington ("The Science and Practice of Pharmacy", Lippincott, Williams & Wilkins, 2000) beschrieben.

In einer Ausführungsform der Erfindung werden die pharmazeutischen Zusammensetzungen, die die erfindungsgemäßen Verbindungen enthalten, oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist. Entsprechende orale Formulierungen sind beispielsweise beschrieben in EP 0 548 356 oder EP 1 126 821.

Geeignete Formulierungen zur raschen Freisetzung von (S)-2-N-Propylamino-5-hydroxytetralin oder dessen pharmazeutisch akzeptablen Salzen und Prodrugs gemäß Anspruch 1 sind insbesondere Formulierungen zur mukosalen Verabreichung, zum Beispiel Bukkal- oder Sublingualdosierungen oder Nasalsprays. Mit solchen Formulierungen können in idealer Weise die unter der L-DOPA-Therapie entstehenden "Talsohlen" der L-DOPA-Konzentration rasch ausgeglichen und die mit diesen "off-phasen" der L-DOPA-Therapie einhergehenden Bewegungsstörungen, z.B. Akinesien, behandelt werden.

Die transmukosale Formulierung kann in fester oder flüssiger Form vorliegen. Feste mukosale Verabreichungsformen sind beispielsweise rasch zerfallende Sublingualtabletten oder mucoadhäsive Darreichungsformen. Bevorzugt werden flüssige Formulierungen, die zur Anwendung als Spray, insbesondere als Nasalspray, geeignet sind.

Eine mukosale Formulierung in Sprayform kann im einfachsten Fall eine Wirkstofflösung sein. Diese kann gegebenenfalls durch Zugabe geeigneter Elektrolyte, z.B. Kochsalz oder Dextrose, isotonisch gemacht worden sein. Ein transmukosales Spray von (S)-2-N-Propylarnino-5-hydroxytetralins oder dessen Prodrugs kann beispielsweise eine wässrige Lösung, eine Lösung in nicht-wässrigen Lösemitteln, wie z.B. in Ölen, Glycerol oder Propylenglykol oder eine Emulsion sein. Ferner kann eine solche transmukosale Formulierung im pharmazeutischen Bereich übliche Puffer enthalten, die den gewünschten pH der Wirkstofflösung einstellen. Vorteilhafterweise wird der pH einer transmukosalen Formulierung so eingestellt, dass die Schleimhäute bei Verabreichung der Formulierung nicht gereizt werden. Dies ist bei nasaler Verabreichung üblicherweise bei einem mild sauren pH im Bereich zwischen 3 und 6 der Fall. Geeignete Puffer sind beispielsweise Acetat-, Citrat- und Phosphatpuffer. Ferner können in der transmukosalen Formulierung, z.B. im Nasalspray, weitere Hilfsstoffe zugegen sein, wie z.B. Löslichkeitsvermittler, Penetrationsverbesserer, Konservierungsmittel, Antioxidanzien, Verdicker und Geschmacksverbesserer.

Ist dagegen eine protrahierte Freisetzung erwünscht, bietet sich eine Formulierung mit retardierter Wirkstofffreisetzung an. Entsprechende orale und nicht-orale Formulierungen sind ebenfalls aus dem Stand der Technik bekannt.

Beispielsweise kann (S)-2-N-Propylamino-5-hydroxytetralin oder dessen Salze oder Prodrugs gemäß Anspruch 1 in Pflasterform auf die Haut des Patienten gebracht werden, wobei der Wirkstoff bevorzugt in einer Matrix aus adhesivem Polymer, z.B. einem selbstklebendem adhesivem Polysiloxan, vorliegt. Beispiele für transdermale Formulierungen finden sich in WO 99/49852, WO 02/89777, WO 02/89778 und WO 2004/012721. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman, Clinical Neuropharmacol. 24, 2001, 163).

Wird dagegen ein Arzneimittel in Form einer subkutanen oder intramuskularen Depotform gewünscht, kann (S)-2-N-Propylamino-5-hydroxytetralin, dessen Salze oder Prodrugs gemäß Anspruch 1 beispielsweise als Salzkristall, z.B. als kristallines Hydrochlorid, in einem hydrophobem, wasserfreiem Medium suspendiert und injiziert werden. Eine Beispielformulierung ist in WO 02/15903 beschrieben.

Alternative pharmazeutische Zubereitungen können beispielsweise Infusions- oder Injektionslösungen, Öle, Suppositorien, Aerosole, Sprays, Pflaster, Mikrokapseln oder Mikropartikel sein.

### Ausführungsbeispiele:

### 1. Bestimmung der Rezeptoraffinitäten

Die Rezeptoraffinitäten wurden durch Verdrängungsexperimente bestimmt. Zu diesem Zweck werden die Rezeptoren mit radioaktiv markierten, rezeptorspezifischen Liganden inkubiert. Überwiegend werden humane Rezeptoren verwendet, die in Zelllinien exprimiert werden. Alternativ finden Membranpräparationen aus Ratten- oder Rinderhirnen Verwendung. Die Inkubationsbedingungen sind publiziert und standardisiert. Den Inkubationsansätzen werden unterschiedliche Konzentrationen der zu testenden Substanz ((S)-2-N-Propylamino-5-hydroxytetralin) zugesetzt, sodass eine Dosis-Bindungskurve erstellt werden kann. Unspezifische Bindung wird von spezifischer Bindung durch Inkubation mit unspezifischen Liganden separiert. Der Anteil spezifischer Bindung bei verschiedenen Substanzkonzentrationen wird in % der maximalen Bindung des Liganden dargestellt. Der lC50-Wert (Konzentration bei 50 %-iger Inhibition der Bindung des Liganden) und die Steigung werden mittels Regressionsanalyse ermittelt. Mittels der Cheng-Pusoff-Gleichung wird der Ki-Wert ermittelt, der dann zum Vergleich herangezogen wird: Je niedriger der Ki-Wert, umso höher die Affinität (siehe Tabelle 1).

### 2. Bestimmung der funktionellen Eigenschaften

Um die intrinsische Aktivität der Substanz zu bestimmen, wurden humane Dopamin-Rezeptoren in Zelllinien (CHO-DUKX-SRE oder SH-SY5Y-SRE) funktionell exprimiert. Das bedeutet, dass nach Bindung eines Agonisten eine intrazelluläre Signalkaskade aktiviert wird, die zur Bildung weiterer Proteine führt. Das Gen eines dieser Proteine, der Luziferase, ist zuvor künstlich insertiert worden. Stimulation der Proteinexpression führt auch zur Bildung von Luziferase, die in Gegenwart von ATP die Emission von Photonen induziert (sog. Lumineszenz), die dann photometrisch gemessen werden kann. Die Intensität der Lumineszenz ist der Stimulation der Rezeptoren proportional. DopaminAgonisten stimulieren die Lumineszenz während Antagonisten keine eigene Wirkung entfalten. Antagonisten inhibieren jedoch die durch Dopamin- oder Agonisten-induzierte Luminszenz. Der Aktivität bei verschiedenen Substanzkonzentrationen wird in % der maximalen Aktivierung durch den endogenen Liganden oder einen geeigneten Agonisten dargestellt. Der EC50-Wert (Konzentration bei 50 %iger Aktivierung) und die Steigung werden mittels Regressionsanalyse ermittelt. Mittels der Cheng-Pusoff-Gleichung wird der Ki-Wert ermittelt, der dann zum Vergleich herangezogen wird: Je niedriger der Ki-Wert, umso höher die Affinität und Aktivität. Für die Wirkung von (S)-2-N-Propylamino-5-hydroxytetralin an Dopamin-Rezeptoren wurden die in Tabelle 2 angegebenen Werte gefunden.

### 3. Ausführungsbeispiel: In vitro Umsetzung eines Prodrugs in die Wirksubstanz

Aus Leberzellhomogenaten von Mensch, Affe, Hund, Ratte oder Maus wird durch differentielle Zentrifugation die Mikrosomenfraktion erhalten, die die wesentlichen metabolisierenden Enzyme enthält; alternativ kann auch die zytoplasmatische Fraktion gewonnen werden. Die subzelluläre Fraktion wird mit einem Puffer so suspendiert, dass eine Lösung mit einem definierten Proteingehalt erhalten wird. Nach Zufügen von 1 µM des zu testenden Prodrugs erfolgt die Inkubation bei 37 °C für 60 min. Anschließend wird (S)-2-N-Propylamino-5-hydroxytetralin mittels HPLC/UV oder auch mittels HPLC/MS quantifiziert und zur eingesetzten Menge in Beziehung gesetzt. Für detailliertere Analysen werden Konzentrations- oder Zeitreihen untersucht.

### 4. Ausführungsbeispiel: Depotsuspension

(a) 1411,2 g Miglyol 812 wird in eine Duran Flasche eingewogen. 14,4 g Imwitor 312 wurde dem Miglyol zugegeben und im Anschluß für 30 Minuten unter Rühren auf 80°C erwärmt. Die klare Lösung wird auf Raumtemperatur abgekühlt und gefiltert.
(b) 1188 g der unter (a) hergestellten Lösung wird in einen Glaslaborreaktor überführt, 12 g Wirkstoff zugesetzt und für 10 Minuten mit einem Ultraturrax bei 10.000 UpM unter Stickstoff homogenisiert. Die Suspension wird bei laufendem Ultraturrax (2.000 UpM) in Braunglasflaschen abgefüllt.

## Patentansprüche

1. Arzneimittel enthaltend (S)-2-N-Propylamino-5-hydroxytetralin, dessen pharmazeutisch akzeptable Salze oder Prodrugs, wobei das Prodrug die allgemeine Formel I aufweist 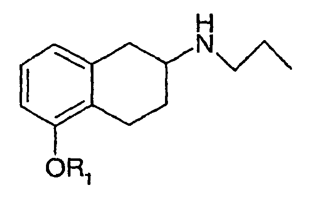 worin R1 ausgewählt ist aus der Gruppe Acyl, Alkoxycarbonyl, Cycloalkoxycarbonyl, Aryloxycarbonyl, Aralkoxycarbonyl, Acetal, Ketal, -C(O)NR2R3, -C(O)NHR2, -P(O₂H)OR2, -P(O₂H)R2,
wobei R2 und R3 jeweils ausgewählt sind aus H, C1-6 Alkyl, C3-10 Cycloalkyl, Benzyl oder Phenyl.

2. Arzneimittel nach Anspruch 1, wobei R1 ausgewählt ist aus C1-6 Alkylcarbonyl, C3-10 Cycloalkylcarbonyl, Benzoyl, -C(O)NR2R3 und -C(O)NHR2.

3. Arzneimittel nach einem der vorhergehenden Ansprüche, wobei das Arzneimittel zur transdermalen, transmukosalen oder parenteralen Verabreichung bestimmt ist.

4. Verwendung von (S)-2-N-Propylamino-5-hydroxytetralin oder dessen pharmazeutisch akzeptable Salze und Prodrugs gemäß einem der Ansprüche 1 oder 2 zur Herstellung eines Arzneimittels zur Behandlung oder Prophylaxe einer Erkrankung ausgewählt aus der Gruppe der Depressionen, krankhaften Angstzustände, sexuelle Dysfunktion, Galactorrhoe, Akromegalie, Glaukoma, kognitive Störungen, Restless Leg Syndrom, Hyperaktivitätssyndrom (ADHS), Hyperprolaktinämie, Hyperprolaktinom, Essstörungen, Dopa-sensitive Dyskinesien, Parkinson-assoziierte Bewegungsstörungen, DOPA- und Neuroleptika-induzierte Bewegungsstörungen, Kokain-, Alkohol-, Opiat- und Nikotinsucht, neurodegenerative Erkrankungen oder zum Abstillen.

5. Verwendung nach Anspruch 4, wobei die Erkrankung ausgewählt ist aus der Gruppe Restless Leg Syndrom, L-DOPA-sensitive Dyskinesien, Parkinson-assoziierte Bewegungsstörungen, L-DOPA- und Neuroleptika-induzierte Bewegungsstörungen sowie Kokain-, Alkohol-, Opiat- und Nikotinsucht.

6. Verwendung nach einem der vorhergehenden Ansprüche, wobei die Erkrankung eine Bewegungsstörung ist, die
(a) Morbus Parkinson-assoziiert,
(b) L-DOPA induziert oder
(c) Neuroleptika-induziert ist.

## Claims

1. Medicament containing (S)-2-N-propylamino-5-hydroxytetralin, pharmaceutically acceptable salts thereof or prodrugs, wherein the prodrug has the general formula I 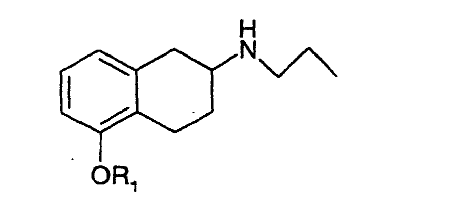 wherein R1 is selected from the group acyl, alkoxycarbonyl, cycloalkoxycarbonyl, aryloxycarbonyl, aralkoxycarbonyl, acetal, ketal, -C(O)NR2R3, -C(O)NHR2, -P(O₂H)OR2, -P(O₂H)R2,
wherein R2 and R3 in each case are selected from H, C1-6 alkyl, C3-10 cycloalkyl, benzyl or phenyl.

2. Medicament according to claim 1, wherein R1 is selected from C1-6 alkylcarbonyl, C3-10 cycloalkylcarbonyl, benzoyl, -C(O)NR2R3 and -C(O)NHR2.

3. Medicament according to one of the preceding claims, wherein the medicament is intended for transdermal, transmucosal or parenteral administration.

4. Use of (S)-2-N-propylamino-5-hydroxytetralin or pharmaceutically acceptable salts thereof and prodrugs according to one of claims 1 or 2 for the production of a medicament for the treatment or prophylaxis of a disease selected from the group of depressions, pathological anxiety states, sexual dysfunction, galactorrhea, acromegalia, glaucoma, cognitive disturbances, restless leg syndrome, hyperactivity syndrome (ADHS), hyperprolactinaemia, hyperprolactinoma, eating disorders, dopa-sensitive dyskinesias. Parkinson-associated motor disturbances. DOPA-induced and neuroleptic-induced motor disturbances, cocaine, alcohol, opiate and nicotine addiction, neurodegenerative diseases or for weaning.

5. Use according to claim 4, wherein the disease is selected from the group restless leg syndrome, L-DOPA-sensitive dyskinesias, Parkinson-associated motor disturbances, L-DOPA-induced and neuroleptic-induced motor disturbances and cocaine, alcohol, opiate and nicotine addition.

6. Use according to one of the preceding claims, wherein the disease is a motor disturbance, which is
(a) Parkinson's disease-associated,
(b) L-DOPA-induced or
(c) neuroleptic-induced.

## Revendications

1. Médicament contenant de la (S)-2-N-propylamino-5-hydroxytétraline, des sels pharmaceutiquement acceptables ou des promédicaments de celle-ci, le promédicament ayant la formule générale 1 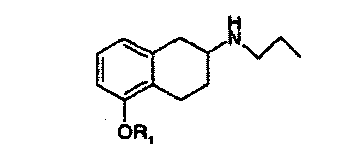 dans laquelle R1 est choisi dans le groupe comprenant acyle, alkoxycarbonyle, cycloalkoxycarbonyle, aryloxycarbonyle, aralkoxycarbonyle, acétal, cétal, -C(O)NR2R3, -C(O)NHR2, -P(O₂H)OR2, -P(O₂H)R2,
dans laquelle R2 et R3 sont choisis chacun parmi H, alkyle en C₁-C₆, cycloalkyle en C₃-C₁₀, benzyle ou phényle.

2. Médicament selon la revendication 1, dans lequel R1 est sélectionné parmi alkylcarbonyle en C₁-C₆, cycloalkylcarbonyle en C₃-C₁₀, benzoyle, -C(O)NR2R3 et -C(O)NHR2.

3. Médicament selon l'une quelconque des revendications précédentes, lequel médicament est destiné à une administration par voie transdermique, transmuqueuse ou parentérale.

4. Utilisation de la (S)-2-N-propylamino-5-hydroxytétraline, des sels pharmaceutiquement acceptables ou des promédicaments de celle-ci selon l'une quelconque des revendications 1 ou 2, pour le traitement ou la prévention d'une maladie choisie dans le groupe composé de : dépressions, anxiété pathologique, troubles des fonctions sexuelles, galactorrhée, acromégalie, glaucome, troubles cognitifs, syndrome des jambes sans repos, syndrome d'hyperactivité (THDA), hyperprolactinémie, prolactinome, troubles de l'alimentation, dyskinésies dopasensibles, troubles moteurs associés à la maladie de Parkinson, troubles moteurs induits par la L-dopa et les neuroleptiques, dépendance à la cocaïne, à l'alcool, aux opiacés et à la nicotine, maladies neurodégénératives, ou pour l'arrêt de la lactation.

5. Utilisation selon la revendication 4, dans laquelle la maladie est choisie dans le groupe composé de : syndrome des jambes sans repos, dyskinésies dopasensibles, troubles moteurs associés à la maladie de Parkinson, troubles moteurs induits par la L-dopa et les neuroleptiques, et dépendance à la cocaïne, à l'alcool, aux opiacés et à la nicotine.

6. Utilisation selon l'une quelconque des revendications précédentes, dans laquelle la maladie est un trouble de la motricité :
(a) associé à la maladie de Parkinson,
(b) induit par la L-dopa, ou
(c) induit par les neuroleptiques.
